Europäisches Patentamt

European Patent Office

Office européen des brevets

(11) Publication number: **0 426 489 A2**

# EUROPEAN PATENT APPLICATION

(21) Application number: 90312028.5

(22) Date of filing: 02.11.90

(51) Int. Cl.⁵: **A61L 2/18**, G02C 7/04

(30) Priority: 03.11.89 US 431641

(43) Date of publication of application:
08.05.91 Bulletin 91/19

(84) Designated Contracting States:
AT BE CH DE DK ES FR GB GR IT LI LU NL SE

(71) Applicant: ALLERGAN, INC.
2525 Dupont Drive, P.O. Box 19534
Irvine, California 92713-9534(US)

(72) Inventor: Gyulai, Peter
1152 Edgeview Drive
Sante Ana, California 92705(US)

(74) Representative: Hutchins, Michael Richard et
al
Graham Watt & Co. London Road Riverhead
Sevenoaks, Kent TN13 2BN(GB)

(54) Hydrogen peroxide destroying compositions and methods of using same.

(57) A composition and method useful for destroying hydrogen peroxide in a liquid aqueous medium, such as that used to disinfect contact lenses, are disclosed. The composition comprises at least one hydrogen peroxide destroying component effective when released into a liquid aqueous medium to destroy or cause the destruction of hydrogen peroxide present in the liquid aqueous medium, and a barrier component acting to substantially prevent the release of the hydrogen peroxide destroying component for a period of time after the composition is initially contacted with a hydrogen peroxide-containing liquid aqueous medium. The barrier component includes a water soluble portion and a water insoluble portion. The hydrogen peroxide destroying component is situated so as to be released into the hydrogen peroxide-containing liquid aqueous medium in response to the dissolving of the water soluble portion into the hydrogen peroxide-containing liquid aqueous medium.

EP 0 426 489 A2

# HYDROGEN PEROXIDE DESTROYING COMPOSITIONS AND METHODS OF USING SAME

## BACKGROUND OF THE INVENTION

This invention relates to hydrogen peroxide destroying compositions, and the use of same, to decrease the concentration of, or even substantially eliminate, hydrogen peroxide present in a liquid medium. More particularly, the invention relates to such compositions which are useful in destroying residual hydrogen peroxide present in a liquid aqueous medium containing a contact lens which has been disinfected by the action of hydrogen peroxide.

Contact lenses should be periodically cleaned and disinfected by the user to prevent infection or other deleterious effects on ocular health which may be associated with contact lens wear. Currently, there are several different conventional systems and methods which enable the user to clean and disinfect their contact lenses between wearing times. These conventional cleaning and disinfection systems can be divided into "hot" and "cold" systems. Hot systems require the use of heat to disinfect the contact lenses, whereas cold systems use chemical disinfectants at ambient temperatures to disinfect the lenses.

Within the realm of cold disinfection systems are hydrogen peroxide disinfection systems. Disinfecting hydrogen peroxide solutions are effective to kill the bacteria, fungi and yeast which may contaminate contact lenses. However, residual hydrogen peroxide on a disinfected contact lens may cause irritation, burning or trauma to the eye unless this hydrogen peroxide is destroyed, i.e., decomposed, neutralized, inactivated or chemically reduced. Therefore, the destruction of the residual hydrogen peroxide in the liquid medium containing the disinfected contact lens is needed to enable safe and comfortable wear of the disinfected contact lens.

Associated with the problem of hydrogen peroxide destruction in contact lens disinfection systems are the problems of easy use and user compliance. To enhance user compliance and ease of use, several efforts have focused on one-step disinfection and hydrogen peroxide destruction. In this regard, various time release tablets containing a core tablet and a totally soluble or insoluble coating have been suggested. Kruse et al U.S. Patent 4,767,559 discloses a one-step contact lens cleaning and disinfecting tablet designed to be totally dissolved in water. A core containing a hydrogen peroxide reducing agent and a catalyst is provided. A jacket mixture containing a hydrogen peroxide generating component is provided and envelopes the core. In this case, the jacket mixture dissolves to form hydrogen peroxide to disinfect the contact lens. Subsequently, a thin lacquer coating surrounding the core of the tablet is dissolved, resulting in the release of the reducing agent and catalyst.

Kay United Kingdom Patent Applicant GB 2 151 039 A discloses a sustained-release tablet composition from which a hydrogen peroxide inactivator, e.g. sodium sulphite, is gradually leached in the presence of a hydrogen peroxide-containing solution used to disinfect contact lenses. The entire tablet, other than the leached hydrogen peroxide inactivator, remains unaffected, i.e., is insoluble in the solution.

Kaspar et al U.S. Patent 4,568,517 discloses a system in which a contact lens is disinfected in an aqueous hydrogen peroxide solution and, after disinfection, the peroxide is reduced by adding a tablet containing a core having a hydrogen peroxide reducing component, e.g. sodium sulfite or sodium thiosulfate, and a coating which slowly totally dissolves in the peroxide solution t release the reducing agent.

A number of other alternatives for a combination tablet or the like containing a hydrogen peroxide sterilizing agent and a hydrogen peroxide reducing agent are disclosed in Schafer et al European Patent Application EP-A-0209071.

Another problem associated with hydrogen peroxide destruction is indicating to the user that peroxide destruction has taken place and/or that peroxide destruction is satisfactorily or acceptably complete (e.g., for safe and comfortable contact lens wearing). Some of the above references do not provide the user with a indication that either peroxide destruction has taken place or is complete while others attempt to address these problems by utilizing a color indicator. The color indicator provides a visual indication of pH change, and indirectly of hydrogen peroxide neutralization. However, some users may develop or have sensitivities to the color indicators or other soluble materials which are solubilized during the disinfection/hydrogen peroxide destruction process.

Accordingly, a need exists for novel, safe and efficacious systems for destroying residual hydrogen peroxide in liquid medium used for cleaning and disinfecting contact lenses.

## SUMMARY OF THE INVENTION

New compositions and methods useful for destroying hydrogen peroxide in a liquid aqueous medium, in particular for destroying residual hydrogen peroxide in a liquid aqueous medium containing a disinfected contact lens, have been discovered. The present invention allows the hydrogen peroxide destroying component or components to be initially contacted with the liquid aqueous medium at the same time the contact lens to be disinfected is initially contacted with liquid aqueous medium. For example, the present compositions and the contact lens to be disinfected can be added to the liquid aqueous medium at substantially the same time. This feature greatly reduces the amount of user time and care required to effectively disinfect his/her lenses and destroy the residual hydrogen peroxide. Better user compliance and a greater degree of user eye safety is provided. The present invention provides a delayed release feature so that the contact lens is effectively disinfected by the action of hydrogen peroxide prior to the release of the hydrogen peroxide destroying component. Also, the present composition is structured so as to provide for positive release of the hydrogen peroxide destroying agent into the medium as opposed to, for example, the system of Kay, described above, in which the peroxide inactivator gradually leaches out of an otherwise totally insoluble tablet. This positive release feature provides assurance that the peroxide destroying component is effectively released into the medium after the lens has been disinfected. This positive release feature is provided without dissolving a large amount of extraneous material into the liquid medium. Such dissolved extraneous material may have adverse affects on the eyes of the lens wearer, and therefore, it is advantageous to minimize the amount of this material which is dissolved. This is in contrast to various of the prior systems, described above, in which all the tablet holding the peroxide destroying component is soluble in the liquid mixture containing the disinfected contact lens.

In one broad aspect, the present invention is directed to a composition including at least one hydrogen peroxide destroying component, and a partially soluble barrier component. The hydrogen peroxide destroying component, hereinafter referred to as HPDC, is effective when released into a liquid aqueous medium to destroy or cause the destruction of hydrogen peroxide present in the liquid aqueous medium. The barrier component acts to substantially prevent the release of the HPDC for a period of time after the composition, preferably in the form of a tablet is initially contacted with a hydrogen peroxide-containing liquid aqueous medium, hereinafter referred to as HPLM. The barrier component includes a water insoluble portion, preferably a polymeric material, which does not dissolve in the HPLM. Also included in the barrier component is a water soluble portion which dissolves over a period of time into the HPLM. The HPDC is situated, e.g., with respect to the barrier component, so as to be released into the HPLM in response to the dissolving of the water soluble portion into the HPLM.

A method for decreasing the concentration of hydrogen peroxide in a HPLM is provided in which a HPLM is contacted with a composition, e.g., as described herein.

Further, a method of disinfecting a lens, preferably a contact lens, is provided. This method includes contacting a lens to be disinfected with a HPLM at effective lens disinfecting conditions to disinfect the lens. The HPLM is also contacted with a composition, e.g., as described herein. This composition preferably produces a liquid aqueous medium having no deleterious concentration of hydrogen peroxide. Thus, after the composition has acted, the disinfected contact lens can be removed from the liquid aqueous medium and placed directly in the eye.

DETAILED DESCRIPTION OF THE INVENTION

The present invention is of value where hydrogen peroxide is used to disinfect all types of lens, e.g., contact lenses, which are benefited by periodical disinfecting. Such lenses, e.g., conventional hard contact lenses and soft contact lenses, may be made of any suitable material or combination of materials and may have any suitable configuration not substantially deleteriously affected by hydrogen peroxide, the present compositions or the present methods.

The present invention is particularly useful for destroying residual hydrogen peroxide in a HPLM which has been used to disinfect a contact lens.

The liquid medium used to disinfect contact lens in the present invention includes a disinfecting amount of hydrogen peroxide. Preferably, a disinfecting amount of hydrogen peroxide means such amount as will reduce the microbial burden by one log unit in three hours. Still more preferably, the hydrogen peroxide concentration is such that the microbial load is reduced by one log order in one hour. Still more preferred are those hydrogen peroxide concentrations which reduce the microbial load by one log unit in 10 minutes or less. Relatively mild aqueous hydrogen peroxide solutions, preferably containing about 0.5% to about 6% of hydrogen peroxide (w/v), are known to be effective disinfecting solutions for contact lenses. These solutions are effective at killing bacteria, fungi and yeast which may be found on contact lenses. However, once a contact lens has been disinfected by being immersed in the HPLM, the residual hydrogen peroxide, e.g., on the lens, should be destroyed so that the lens may be safely and comfortably worn on the eye. If this residual hydrogen peroxide is not destroyed before the lens is worn, irritation to the eye or wearing discomfort may occur.

Thus, the present compositions, which are preferably initially contacted with the HPLM at substantially the same time as the contact lens to be disinfected, allow for effective lens disinfection and, in addition, effectively destroy the residual hydrogen peroxide remaining in the HPLM so that the disinfected lens can be removed from the liquid medium and placed directly into the eye for safe and comfortable wear. The present composition is preferably present in the form of a tablet. The composition may be present in the form of at least one item, e.g., tablet, which includes a core layer and a coating layer. The coating layer includes the barrier component and preferably substantially surrounds the core layer, which includes the HPDC.

Any suitable HPDC may be included in the present compositions. Such HPDCs should effectively destroy the residual hydrogen peroxide and have no undue detrimental effect on the disinfected lens or on the eye into which the disinfected lens is placed. Among the useful HPDCs are hydrogen peroxide reducing agents, peroxidases (meaning to include therein catalase) and mixtures thereof.

Examples of the hydrogen peroxide reducing agents which are useful in the present invention are alkali metal in particular sodium, thiosulfates; thiourea; alkali metal, in particular sodium, sulfites; thioglycerol; N-acetylcysteine alkali metal, in particular sodium, formiates; ascorbic acid; isoascorbic acid; glyoxylic acid; mixtures thereof and the like. A particularly useful peroxidase is catalase. The peroxidases, and especially catalase, are very beneficial in the present invention since such HPDCs are effective to substantially eliminate hydrogen peroxide from a liquid medium in a reasonable period of time, e.g., on the order of about 5 minutes to about 12 hours, preferably about 3 hours to about 6 hours, after the HPDC is initially released into the HPLM.

The amount of HPDC employed is preferably sufficient to destroy all the hydrogen peroxide present in the HPLM into which the HPDC is placed. Excess HPDC may be employed. For example, an excess of HPDC of up to about 40% or more of that amount of HPDC required to destroy all the hydrogen peroxide present in the HPLM may be employed. Larger excesses of HPDC are to be avoided since the HPDC itself may cause problems with the disinfected contact lens and/or the ability to safely and comfortably wear such disinfected contact lens. When catalase is employed as a HPDC, it is preferably present in an amount of about 150 to about 200 units of catalase activity/percent (w/v) of hydrogen peroxide in the HPLM/ml of HPLM. For example, an especially useful amount of catalase for use in an aqueous solution containing about 3% (w/v) hydrogen peroxide is about 520 units of catalase activity/ml of solution.

The HPDC may be combined with one or more other components, e.g., in the core of a layered tablet. Such other components may include, for example, fillers, binders, tonicity agents, contact lens conditioning/wetting agents, buffering agents, lubricating agents and the like. Each of these components may be present, if at all, in an amount effective to perform its designated function or functions. Examples of each of these types of components are conventional and well known in the art. Therefore, a detailed description of such components is not presented here. An illustrative HPDC-containing core tablet may have the following composition:

|  | Wt.% |
| --- | --- |
| HPDC | 1-30 |
| Filler | 15-90 |
| Tonicity Agent | 1-30 |
| Buffer | 2-50 |
| Lubricating Agent | 0-30 |

In a particularly useful embodiment, th HPDC is combined with at least one enzyme effective to remove protein-based debris from a contact lens.

The enzyme employed may be selected from peroxide-active enzymes which are conventionally employed in the enzymatic cleaning of contact lenses. For example, many of the enzymes disclosed in Huth et. al. U.S. Patent 4,670,178 and Karageozian et al U.S. Patent 3,910,296 are useful in the present invention. These patents are incorporated in their entirety by reference herein. Among the useful enzymes are those selected from proteolytic enzyme and mixtures thereof.

Preferred proteolytic enzymes are those which are substantially free of sulfhydryl groups or disulfide bonds, whose presence may react with the active oxygen in the HPLM to the detriment of the activity of the enzyme. Metalloproteases, those enzymes which contain a divalent metal ion such as calcium, magnesium or zinc bound to the protein, may also be used.

A more preferred group of proteolytic enzymes are the serine proteases, particularly those derived from Bacillus and Streptomyces bacteria and Asperigillus molds. Within this grouping, the still more preferred enzymes are the derived alkaline proteases generically called subtilisin enzymes. Reference is made to Deayl, L., Moser, P.W. and Wildi. B.S., "Proteases of the Genus Bacillus ". II alkaline Proteases, "Biotechnology and Bioengineering, Vol. XII, pp 213-249 (1970) and Keay, L. and Moser, P.W., "Differentiation of Alkaline Proteases form Bacillus Species" Biochemical and Biophysical Research Comm., Vol 34, No. 5, pp 600-604, (1969).

The subtilisin enzymes are broken down onto two subclasses, subtilisin A and subtilisin B. In the subtilism A grouping are enzymes derived from such species are B. subtilis , B. licheniformis and B. pumilis . Organisms in this sub-class produce little or no neutral protease or amylase. The subtilisin B sub-class is made up of enzymes from such organisms a B. subtilis , B. subtilis var. amylosacchariticus , B. amyloliquefaciens and B. subtilis NRRL B3411. These organisms produce neutral proteases and amylases on a level about comparable to their alkaline protease production. One or more enzymes from the subtilisin A sub-class are particularly useful.

In addition other preferred enzymes are, for example, pancreatin, trypsin, collaginase, keratinase, carboxylase, aminopeptidase, elastase, and aspergillopeptidase A and B, pronase E (from S. griseus) and dispase (from Bacillus polymyxa).

An effective amount of enzyme is to be used in the practice of this invention. Such amount will be that amount which effects removal in a reasonable time (for example overnight) of substantially all proteinaceous deposits from a lens due to normal wear. This standard is stated with reference to contact lens wearers with a history of normal pattern of protein accretion, not the very small group who may at one time or another have a significantly increased rate of protein deposit such that cleaning is recommended every day, or every two or three days.

The amount of enzyme required to make an effective cleaner will depend on several factors, including the inherent activity of the enzyme, and the extent of its interaction with the hydrogen peroxide present.

As a basic yardstick, the working solution should contain sufficient enzyme to provide about 0.001 to about 3 Anson units of activity, preferably about 0.01 to about 1 Anson units, per single lens treatment. Higher or lower amounts may be used.

Enzyme activity is pH dependent so for any given enzyme, there is a particular pH range in which that enzyme will functions best. The determination of such range can readily be done by known techniques.

The barrier component of the present composition includes a water soluble portion and a water insoluble portion. That is, part of the barrier component is soluble in a liquid aqueous medium, such as the HPLM used to disinfect contact lenses, at normal use conditions and part of the barrier component is insoluble in such a liquid aqueous medium at normal use conditions. In one useful embodiment, the water soluble portion is randomly distributed, e.g., as a separate solid phase in the barrier component. The soluble portion is preferably situated so as to provide or create pores in the barrier component as it dissolves into a liquid aqueous medium, e.g., a HPLM. These pores act as channels which expose the HPLM to the action of the HPDC so that the hydrogen peroxide in the HPLM is destroyed.

The soluble portion of the barrier component may be made of any suitable material or combination of materials. Such material or materials preferably are such that their presence in the liquid aqueous medium containing the disinfected contact lens has no substantial detrimental effect on the lens itself, on the ability of the HPDC to destroy hydrogen peroxide, or on the ability to place the disinfected lens directly into the eye for safe and comfortable wear. Examples of materials useful for the soluble portion of the barrier component include sucrose, sodium chloride, potassium chloride, and mixtures thereof. An especially useful material as the soluble portion is sucrose.

The insoluble portion of the barrier component may be made of any suitable material or combination of materials, provided that such material or materials do not have any substantial detrimental effect on the lens itself, on the ability of the HPDC to destroy hydrogen peroxide or on the ability to place the disinfected lens directly in the eye for safe and comfortable wear. The water insoluble portion of the barrier component is preferably made of a polymeric material. The polymeric material is preferably derived from one or more, e.g., two or three, vinyl functional monomers. Examples of vinyl functional monomers include vinyl chloride and vinyl acetate.

A particularly useful polymeric material for use in the insoluble portion of the barrier component is a terpolymer of polyvinylchloride, preferably about 80% to about 95% by weight; polyvinylacetate, preferably about 0.5% to about 19% by weight; and polyvinylalcohol, preferably about 0.5% to about 10% by weight.

5

Although the make-up of the terpolymer is expressed as a mixture of polymers, it is understood that the terpolymer is made form individual monomers, e.g. vinyl chloride and vinyl acetate with a portion of the acetate groups on the polymer being coverted to hydroxyl groups using conventional techniques.

The relative amount of water soluble portion present in the barrier component may be varied depending, for example, on the desired properties of the composition. Thus, if it is desired to have relatively rapid release of the HPDC, a relatively high concentration of soluble portion can be used. The soluble portion is preferably present in an amount in the range of about 10% to about 97% by weight of the total soluble portion and insoluble portion present in the barrier component.

One particularly useful barrier component for use in the present invention is described in Lindahl et al U.S. Patent 4,557,925, which is hereby incorporated in its entirety by reference herein.

An additional embodiment of the present invention provides for a composition in which at least one enzyme capable of removing protein-based debris from a contact lens, as described elsewhere herein, is included separate and apart from the HPDC. Preferably, in this embodiment, the composition is present in the form of at least one item, e.g., a tablet, comprising a core layer, a first coating layer and a second coating layer. The second coating layer includes the enzyme, the first coating layer includes the barrier component, and the core layer includes the HPDC. The second coating layer preferably substantially surrounds the first coating layer, which, preferably, substantially surrounds the core layer. Using this embodiment, the contact lens is preferably cleaned of protein-based debris at the same time the contact lens is disinfected.

Briefly, the two and three layer tablet can be prepared as follows. The HPDC-containing material is formed into a tablet, e.g., using conventional tableting techniques. The insoluble portion of the barrier component is dissolved in a suitable solvent and the soluble portion of the barrier component is combined therewith, e.g., to form a suspension containing this soluble portion. This suspension is applied to the HPDC-containing tablet. The coating is dried and a coated, or two layer, tablet is produced.

As noted above, one or more enzymes useful to remove protein-based debris from a contact lens can be included with the HPDC or can be located in a third layer. Conventional techniques can be employed to place a third layer containing such enzyme or enzymes on the two layer tablet produced as discussed previously.

The present method of disinfecting a lens, preferably a contact lens, includes contacting the lens to be disinfected with a HPLM at effective lens disinfecting conditions. The HPLM is contacted with a composition which includes at least one HPDC and a barrier component, such as described herein. Using this method, the lens is disinfected and the residual hydrogen peroxide in the HPLM is effectively destroyed. Thus, after the HPDC has been released into the HPLM and acts to effectively destroy the residual HPDC, the lens can be safely and comfortably taken directly from the liquid medium in which it was disinfected.

In a particularly useful embodiment, the contact lens to be disinfected is placed into the HPLM at substantially the same time as in the HPDC/barrier component- containing-composition. After a predetermined period of time, during which the contact lens is disinfected, the HPDC is released into the HPLM and effectively destroys the residual hydrogen peroxide.

In the event that a protein-based debris removing enzyme is present in the composition, the contact lens in the liquid medium is also effectively cleaned of any protein-based debris. This cleaning action can occur either at the time the lens is being disinfected, e.g., if the enzyme is released into the HPLM when the composition is initially contacted with the HPLM or shortly thereafter; or after the lens is disinfected, e.g., if the enzyme is released into the HPLM when the HPDC is released into the HPLM or thereafter.

It is preferred that the HPDC not be released into the HPLM until the lens has been immersed in the HPLM for a time sufficient, more preferably in the range of about 1 minute to about 4 hours and still more preferably in the range of about 5 minutes to about 1 hour, to effectively disinfect the lens. It is also preferred that substantially all of the residual hydrogen peroxide in the HPLM be destroyed in less than about 12 hours, more preferably in less than about 6 hours and still more preferably in less than about 4 hours, after the HPDC is initially released into the HPLM.

The disinfecting contacting preferably occurs at a temperature to maintain the liquid medium substantially liquid. For example, when the liquid medium is aqueous-based, it is preferred that the contacting temperature be in the range of about 0°C. to about 100°C., and more preferably in the range of about 10° C. to about 60° C. and still more preferably in the range of about 15° C. to about 30° C. Contacting at or about ambient temperature is very convenient and useful. The contacting preferably occurs at or about atmospheric pressure. This contacting preferably occurs for a time to substantially completely disinfect the lens being treated. Such contacting times can be in the range of about 1 minute to about 12 hours or more.

EXAMPLE 1

A two layer tablet, having a core tablet surrounded by a coating layer was prepared for testing. The core tablet had the following compositions:

CORE TABLET

| | | |
|---|---|---|
| Crystalline catalase[1] | 1.5 | mg |
| Sodium chloride | 89.4 | mg |
| Dibasic sodium phosphate (anhydrous) | 12.0 | mg |
| Monobasic sodium phosphate monohydrate | 1.0 | mg |
| Polyethylene glycol (molecular weight of about 3350) | 1.0 | mg |

COATING LAYER [2]

Insoluble portion
Soluble portion
} 8.3 mg

(1) The amount of catalase added was determined by an assay of the batch of product to be used. The tablets prepared each contained at least 3000 units of catalase activity, but no more than 7280 units of catalase activity.

(2) About 40% by weight of the coating layer was the soluble portion made of sucrose and was randomly distributed throughout the coating layer. The insoluble portion included a terpolymer of polyvinylchloride, polyvinylacetate and polyvinylalcohol.

The coating layer was prepared and applied to the core tablet substantially in accordance with the teachings of Lindahl et al U.S. Patent 4,557,925.

A series of sixty (60) two layer tablets having the above composition were tested to determine the effectiveness of these tablets in destroying hydrogen peroxide. This test was conducted as follows. 10 ml of a 3% (w/v) aqueous solution of hydrogen peroxide was provided at room temperature. The two layer tablet was introduced into the solution and periodic determinations of hydrogen peroxide concentration were made.

Results of these tests were as follows:

| Time to destroy 20% of the hydrogen peroxide | 44 ± 17 minutes |
| Time to destroy sufficient hydrogen peroxide to provide a solution containing less than 10 ppm (w/v) of hydrogen peroxide | 125 ± 39 minutes |

These results demonstrate that the coating layer effectively delays the release of the catalase for a time sufficient to allow the action of the hydrogen peroxide in the aqueous solution to effectively disinfect a contact lens which is introduced into the solution at the same time as the two layer tablet is introduced. Further, these results also demonstrate that the hydrogen peroxide in the solution can be substantially completely destroyed by the catalase in a reasonable time so that a disinfected contact lens can be removed from this solution and placed directly onto a human eye for safe and comfortable wear.

### EXAMPLE 2

A two layer tablet in accordance with Example 1 is used to disinfect a conventional soft contact lens as follows. 10 ml of a 3% (w/v) aqueous solution of hydrogen peroxide is provided at room temperature. The contact lens to be disinfected and the two layer tablet are placed in the solution at the same time. For approximately one-half hour, the solution remains substantially quiet, i.e., substantially no bubbling (gas solution) takes place. For the next approximately two hours, the solution bubbles. After this period of time, the solution becomes and remains quiet. Four hours after the contact lens is first introduced into the solution, it is removed from the solution and placed directly into the wearer's eye. It is found that after four hours, the contact lens is effectively disinfected. Also, the lens wearer experiences no discomfort or eye irritation from wearing the disinfected contact lens. The bubbling of the solution provides an indication that hydrogen peroxide destruction is occurring. An indication that the peroxide destruction is complete is provided when the bubbling stops.

### EXAMPLE 3

A two layer tablet is prepared as in Example 1 except that sufficient subtilisin A is included in the core tablet to provide the core tablet with 40 ppm (by weight) of this enzyme.

This enzyme containing tablet is used to disinfect and clean a protein-based debris laden soft contact lens as follows. 10 ml of a 3% (w/v) aqueous solution of hydrogen peroxide is provided at room temperature. The contact lens to be disinfected and cleaned and the enzyme-containing two layer tablet are placed in the solution at the same time. For approximately one-half hour the solution remains substantially quiet. For the next approximately two hours, the solution bubbles. After this period of time, the solution becomes and remains quiet. 10 hours after the contact lens is first introduced into the solution, it is removed from the solution and placed directly into the wearer's eye. It is found that after 10 hours, the contact lens is effectively disinfected and cleaned of protein-based debris. The lens wearer experiences no discomfort or eye irritation from wearing the disinfected and cleaned contact lens.

### EXAMPLE 4

A two layer table is prepared as in Example 1, and is further spray coated with an aqueous mixture of polyvinylpyrrolidone, sodium carbonate and subtilisin A. The spray coated tablet is dried. The amount of subtilisin A is selected to provide 40 ppm by weight of this enzyme based on the weight of the core tablet.

This enzyme-containing tablet is used to disinfect and clean a protein-based debris laden soft contact lens substantially as described in Example 3. 10 hours after the contact lens is first introduced into the solution, it is removed from the solution and placed directly into the wearer's eye. It is found that after 10 hours, the contact lens is effectively disinfected and cleaned of protein-based debris. The lens wearer experiences no discomfort or eye irritation from wearing this disinfected and cleaned contact lens.

While this invention has been described with respect to various specific examples and embodiments, it is to be understood that the invention is not limited thereto and that it can be variously practiced within the scope of the following claims.

As will be appreciated from the foregoing, the invention also comprises a process for preparing the peroxide-decomposing composition, which process comprises bringing the hydrogen peroxide-destroying component into association with the barrier component and optionally any other components, for example the proteolytic enzyme. For example, the invention comprehends a process wherein the hydrogen peroxide-destroying component is coated with the barrier component, e.g. to form a tablet having two or more layers.

## Claims

1. A composition comprising at least one hydrogen peroxide destroying component effective when released into a liquid aqueous medium to destroy or cause the destruction of hydrogen peroxide present in the liquid aqueous medium, and a barrier component acting to substantially prevent the release of said hydrogen peroxide destroying component for a period of time after said composition is initially contacted with a hydrogen peroxide-containing liquid aqueous medium, said barrier component including a water soluble portion and a water insoluble portion, and said hydrogen peroxide destroying component being situated so as to be released into said hydrogen peroxide-containing liquid aqueous medium in response to the dissolving of said water soluble portion into said hydrogen peroxide-containing liquid aqueous medium.

2. The composition of claim 1 wherein said composition is structured so that said hydrogen peroxide destroying component is released into said hydrogen peroxide-containing liquid aqueous medium at a predetermined time after said composition is initially contacted with said hydrogen peroxide-containing liquid aqueous medium.

3. The composition of claim 1 wherein said composition (for example a tablet) is in the form of at least one item comprising a first layer and a second layer, said first layer including said hydrogen peroxide destroying component, and said second layer including said barrier component.

4. The composition of claim 3 wherein said second layer substantially surrounds said first layer.

5. The composition of any one of claims 1 to 4 wherein said hydrogen peroxide destroying component is a hydrogen peroxide reducing agent; or a peroxidase such as catalase; or a mixture thereof.

6. The composition of any one of the preceding claims wherein said hydrogen peroxide destroying component is selected from the group consisting of alkali metal thiosulfates, thiourea, alkali metal sulfites, thioglycerol, N-acetylcysteine, alkali metal formiates, ascorbic acid, isoascorbic acid, glyoxylic acid and mixtures thereof.

7. The composition of any one of the preceding claims wherein said water soluble portion is situated so as to create pores in said barrier component upon dissolving into said hydrogen peroxide-containing liquid aqueous medium.

8. The composition of any one of the preceding claims wherein said water soluble portion is distributed randomly in said barrier component.

9. The composition of any one of the preceding claims wherein said water soluble portion is sucrose; or sodium chloride; or potassium chloride or a mixture of any of the aforesaid substances.

10. The composition of claim 1 wherein said water insoluble portion is made of a polymeric material, for example a polymeric material derived from at least one vinyl functional monomer; suitably at least two vinyl functional monomers.

11. The composition of any one of the preceding claims wherein said water insoluble portion is made of a polymeric material selected from the group consisting of terpolymers of polyvinylchloride, polyvinylacetate and polyvinylalcohol and mixtures thereof.

12. The composition of claim 11 wherein said terpolymers include about 80% to about 95% by weight polyvinylchloride, about 0.5% to about 19% by weight of polyvinylacetate an about 0.5% to about 10% by weight of polyvinylalcohol.

13. The composition of any one of the preceding claims wherein said water soluble portion is present in an amount in the range of about 10% to about 97% by weight of the total of said soluble portion and said insoluble portion.

14. The composition of any one of the preceding claims which further comprises at least one enzyme (e.g. a peroxide active enzyme such as an enzyme of the Subtilisin A class) capable of removing protein-based debris from a contact lens in an amount effective to substantially remove the protein-based debris from a protein-based debris laden contact lens, said composition being structured so that upon introduction into said hydrogen peroxide-containing liquid aqueous medium said enzyme is released before or at substantially the same time as said hydrogen peroxide destroying component is released, said composition for example being in the form of at least one item comprising a core layer, a first coating layer and a second coating layer, said second coating layer including said enzyme, said first coating layer including said barrier component, and said core layer including said hydrogen peroxide destroying component.

15. A method of decreasing the concentration of hydrogen peroxide in a liquid aqueous medium comprising:

contacting a hydrogen peroxide-containing liquid aqueous medium with a composition as defined in any one of claims 1 to 14.

16. A method of disinfecting a lens comprising:

contacting a lens to be disinfected with a hydrogen peroxide-containing aqueous liquid medium at effective lens disinfecting conditions, thereby disinfecting said lens; and

contacting said hydrogen peroxide-containing aqueous liquid medium with a composition as defined in any one of claims 1 to 14.

17. The method of claim 16 wherein the composition contains an enzyme as defined in claim 14 and wherein protein-based debris is removed from said contact lens to be disinfected at substantially the same time said contact lens to be disinfected is being disinfected.